# EUROPEAN PATENT APPLICATION

(11) **EP 2 110 145 A1**
(43) Date of publication of application: **21.10.2009**
(21) Application number: 09005340.6
(22) Date of filing: 15.04.2009
(51) Int. Cl.: A61L 9/20, B01D 53/86, F24F 3/16, F24C 15/20

(54) **Air purification system and method in particular of a domestic environment**

(30) Priority: 16.04.2008 IT MI20080690
(71) Applicant: BARALDI SRL, 20030 Senago (MI) (IT)
(72) Inventor: Baraldi, Lorenzo, 20030 Senago (MI) (IT)
(74) Representative: Carone, Marco

(57) **Abstract**

Air purification system 10 preferably of a domestic environment comprising a plurality of metallic or polymeric elements 20 coated with at least a substance preferably antibacterial comprising titanium dioxide and/or silver ion which are activated through UV rays in such a way to oxidize and/or transform organic elements and/or odour emissions and/or toxic air pollutants purifying consequently said air.

The purification system 10 besides comprises at least an internal light source 25 of UV rays having a wavelength inferior to 400 nm which is able to activate said at least a substance in order to catalyze and accelerate the purification of the air.

Air purification method preferably of a civil environment, in particular of a domestic environment, comprising a phase of a) illuminate at least a substance preferably antibacterial comprising titanium dioxide and silver ion with UV rays having a wavelength inferior to 400 nm obtaining at least an activated substance, and by comprising a phase of b) force an air flow preferably at low speed on the at least an activated substance obtaining a purified air through photocatalysis.

## Description

The present invention refers to an air purification system and method in particular of a domestic environment.

The purification systems of a domestic environment can be identified through two typology: extractor hood in the kitchen room or filtering systems in the other rooms.

The extractor hood work through an electric motor and a fan which extract the air in proximity of the cocking zone and which make flow the same out of the room through an evacuation conduit connected to an external environment.

This can imply a thermal dissipation of the kitchen environment.

Instead a filtering hood bring the cooking greases through a mechanic filtering and odours through the activated carbon filters, before to put again the air in the kitchen environment.

Both the versions imply the use of an high speed electric motor for moving an elevated quantity of air, which cause noisy rumours.

Traditional air purification systems need of frequently cleaning and washing of the filtering parts which tend to lose their filtering property when them tend to saturated, consequently determining the disadvantage of high maintenance and substitution costs of the active filtering parts in order to not compromise the functioning of the systems.

Purpose of the present invention is to realize an air purification system and method in particular of a domestic environment which permit to purify completely the air from various elements in suspension in the same, increasing the purification efficiency and drastically reducing times and costs for maintenance.

This purpose according to the present invention is achieved by realizing an air purification system and method as shown in claims 1 and 12.

Further features of the invention are pointed out in the following claims.

The features and the advantages of an air purification system and method in particular of a domestic environment according to the present invention will appear more evident from the following, illustrative and not limitative description, referred to the attached schematic drawings in which:

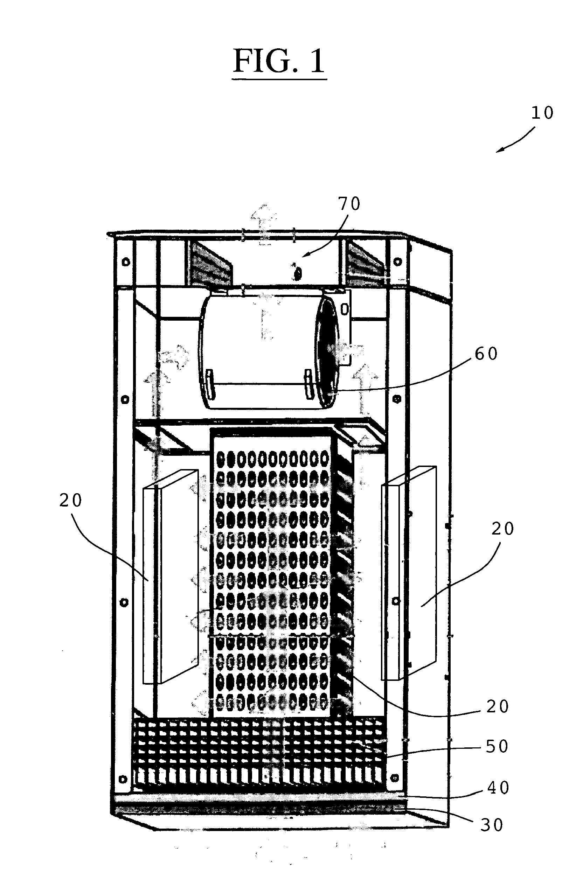

figure 1 is a raised frontal schematic view of a preferred form of embodiment of an air purification system in particular of a civil or domestic environment according to the present invention;

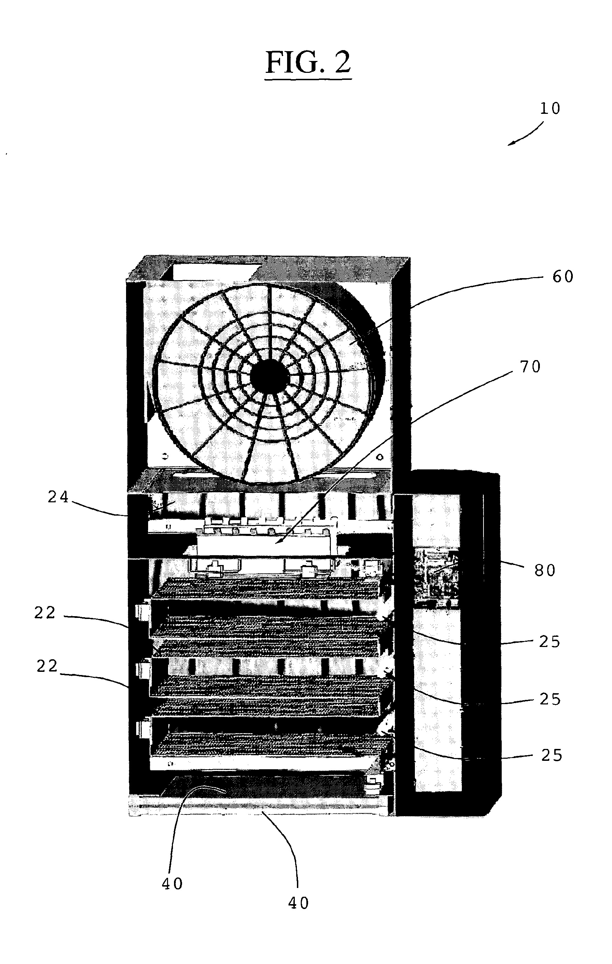

figure 2 is a raised frontal partially sectioned schematic view of another preferred form of embodiment of an air purification system in particular of a civil or domestic environment according to the present invention;

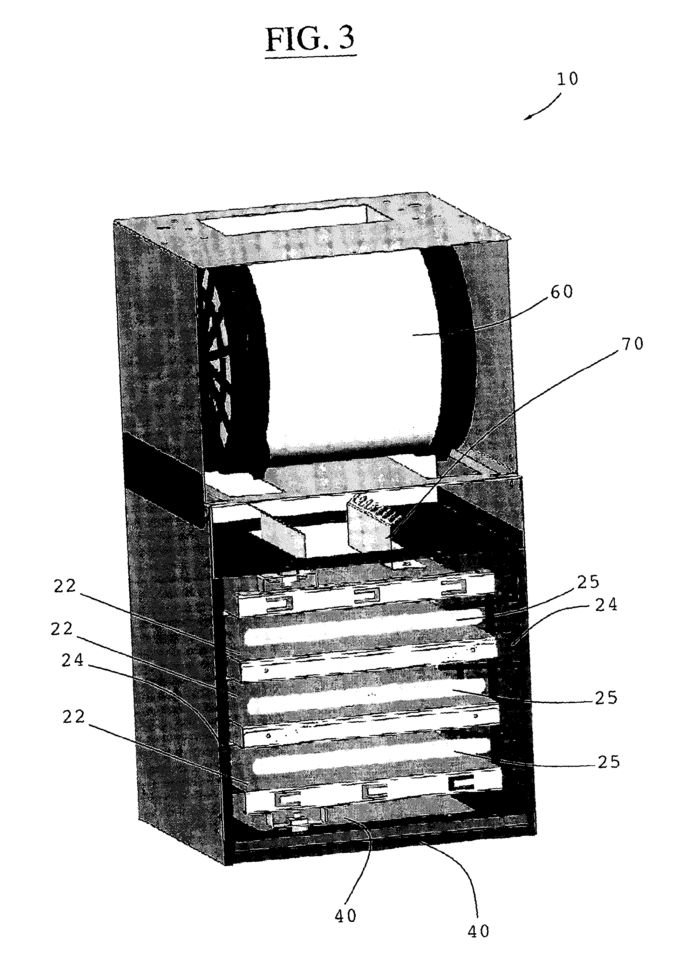

figure 3 is a raised frontal partially sectioned schematic view of another preferred form of embodiment of an air purification system in particular of a civil or domestic environment according to the present invention.

With reference to the figures, it is shown an air purification system 10 preferably of a domestic environment comprising a plurality of metallic or polymeric elements 20 coated with at least a substance preferably antibacterial comprising titanium dioxide and/or silver ion which are activated through UV rays in such a way to oxidize and/or transform a plurality of organic elements and/or odour emissions and/or toxic air pollutants, purifying consequently said air.

Preferably each element 20 of said plurality of metallic and/or polymeric elements 20 comprises a thin coating film comprising titanium dioxide and/or silver ion in particular in form of aggregated nanoparticles, each of which shows preferably a dimension included between 10 and 100 nm, which can be applied through spray with special micronized nozzles in such a way to coat on the external surface of said plurality of elements a thin transparent film of activated substance, in various concentrations and breathable.

Advantageously in this way it is obtained a very high activated surface which permit to obtain a much elevated purification efficiency and at the same time a great reduction of volume of said air purification system 10.

In particular said at least a substance preferably antibacterial activated through UV rays permit to treat and to purify said air from:
- organic elements of various type such as bacteriums, mushroom ventilators and spores;
- odour emissions, as phenols and poly phenols (food odours);
- toxic air pollutants;
- microscopic particles as in particular fine particles, PM10, PM2,5.

Preferably said thin coating film, preferably activated through UV rays, shows a thickness inferior to 0,3 mm and in particular inferior to 0,1 mm.

Preferably said plurality of metallic and/or polymeric elements 20 comprises at least a polymeric element 24 preferably a polymeric foam, in particular a PVC foam, which is coated with titanium dioxide, and in particular at least a metallic net preferably of aluminium or steel which is also coated with titanium dioxide, for achieving a great surface able to photo catalyze the purification of said air.

The present invention is an apparatus which works through the passage of the air in a system comprising a plurality of metallic or polymeric elements 20 chemically treated through substances containing titanium dioxide and/or silver ion.

The apparatus comprises an internal light source which produce ultraviolet light with a wavelength inferior to 400 nm.

In other terms said air purification system 10 comprises at least an internal light source 25 of UV rays having a wavelength inferior to 400 nm which is able to activate said at least a substance in order to catalyze and accelerate the purification of the air.

Preferably said at least an internal light source 25 of UV rays is able to produce UV rays having a wavelength preferably included between 400 nm and 315 nm or preferably included between 315 nm e 280 nm.

The at least an internal UV light source 25 can have different power level, depending of the dimensions of the environment and of the applications.

The purification of the air is activated through a process of photo assisted catalysis.

The substances presents in the air come in the air purification system 10 which oxidize or mineralize the organic compound, transforming them in little quantity of simple mineral salts (sodium Ion, ion), while the suspended particles are retained internally to the air purification system 10 and disaggregated or transformed in hydrogen peroxide.

The air purification system 10 in particular permit the passage of the air in more sections, in other terms the air purification system 10 preferably comprises a plurality of filtering chambers (30,40,50), each of which comprises a multilayer metallic filter 30 in particular in aluminium preferably coated with TiO₂, and/or a carbon activated based filter 40 as in particular a granular carbon activated filter, or a filter in non-woven fabric impregnated with activated carbon or an acrylic filter impregnated with activated carbon.

According to a preferred form of embodiment said air purification system 10 comprises at least an electrostatic filter 50 for remove microscopic particles which in particular fine particles, PM10, PM2,5.

According to a preferred form of embodiment said air purification system 10 comprises an air ionizer 70 integrated in the same for enrich the air of negative ion before to put the same again in said environment.

The air purification system 10 treat the air through a passage at low speed which permit to the same to come in contact with the surfaces of said metallic or polymeric elements 20 treated with said at least a substance preferably antibacterial activated the photocatalysis.

Preferably said air purification system 10 comprises ventilation means as an electric motor 60 at low speed, which suck the air of the environment making come in contact with said metallic or polymeric elements 20 coated with said at least a substance preferably antibacterial.

Preferably said air purification system 10 comprises electronic control means 80 comprising in particular an electronic board 80, which are able to control preferably said ventilation means and said at least an internal UV light source 25 and preferably an air ionizer 70.

Preferably said air purification system 10 comprises a command interface comprising preferably a plurality of buttons and in particular also a display for control preferably said electronic control means 80.

According to a further aspect of the present invention it is furnished an household appliance comprising an air purification system 10 according any one of the form of embodiment previously described which is able to purify said air and to put again the same in said environment preferably domestic.

According to a further aspect of the present invention it is furnished a filtering hood for a domestic environment in particular of a kitchen comprising an air purification system 10 according any one of the form of embodiment previously described.

Said filtering hood is preferably integrated with said air purification system 10 and is able to purify said air and to put again the same in said environment preferably domestic, in other terms said filtering hood is an air recirculation filtering hood.

According to a further aspect of the present invention it is furnished an air purification method preferably of a civil environment, in particular of a domestic environment, in particular through an air purification system comprising a plurality of metallic and/or polymeric elements 20 coated with at least a substance preferably antibacterial comprising titanium dioxide and silver ion.

Said air purification method comprises a phase of a) illuminate at least a substance preferably antibacterial comprising titanium dioxide and silver ion with UV rays having a wavelength inferior to 400 nm obtaining at least an activated substance, and besides comprising a phase of b) force an air flow preferably at low speed on said at least an activated substance obtaining a purified air through photocatalysis.

Advantageously this permit to purify said air and to avoid substitution and maintenance operations of said at least a substance preferably antibacterial, which in the form of a thin breathable film permit to maintain its catalytic property for extremely long time.

Preferably said air purification method comprises besides a phase of c) filtering and/or ionizing said purified air.

Advantageously this permit to remove dusts in particular fine which for example PM10 particles and/or others odours and/or toxics substances as previously described.

Preferably said air purification method comprises a phase of d) put the purified air in said environment, permitting advantageously a purification and a recirculation of the air for the time necessary to the purification of the same.

Advantageously a air purification system and an air purification method preferably of a domestic environment according to the present invention do not need of conduits connected to an external environment, avoiding thermal dissipations and achieving therefore a good energy saving.

Besides the air purification system and method of the present invention, being much efficient and making flow the air with a low speed, they permit a great reduction of the noisy emissions respect to the traditional systems.

Finally the long life cycle of said at least a active catalytic substance, containing titanium dioxide and silver ion, preferably in form of aggregated nanoparticles and/or in form of a thin coating applied on said plurality of metallic and/or polymeric elements, permit a reduction of the maintenance operations, and therefore a reduction of costs.

## Claims

1. Air purification system (10) preferably of a domestic environment **characterized by** comprising a plurality of metallic and/or polymeric elements (20) coated with at least a substance preferably antibacterial comprising titanium dioxide and/or silver ion which are activated through UV rays in such a way to oxidize and/or transform a plurality of organic elements and/or odour emissions and/or toxic air pollutants, purifying consequently said air.

2. Air purification system (10) according to claim 1, **characterized in that** each element (20) of said plurality of metallic and/or polymeric elements (20) comprises a thin coating film comprising titanium dioxide and/or silver ion in particular in form of aggregated nanoparticles.

3. Air purification system (10) according to claim 1 or 2, **characterized by** comprising at least an internal light source (25) of UV rays having a wavelength inferior to 400 nm which is able to activate said at least a substance in order to catalyze and accelerate the purification of the air.

4. Air purification system (10) according any one of the claims from 1 to 3, **characterized by** comprising a plurality of filtering chambers (30,40,50), each of which comprises a multilayer metallic filter (30) in particular in aluminium preferably coated with TiO₂, and/or a carbon activated based filter (40) as in particular a granular carbon activated filter, or a filter in non-woven fabric impregnated with activated carbon or an acrylic filter impregnated with activated carbon.

5. Air purification system (10) according any one of the claims from 1 to 4, **characterized by** comprising at least an electrostatic filter (50).

6. Air purification system (10) according any one of the claims from 1 to 5, **characterized by** comprising an air ionizer (70) integrated in the same.

7. Air purification system (10) according any one of the claims from 1 to 6, **characterized by** comprising low speed ventilation means (40) which for example electric motor (60) at low speed.

8. Air purification system (10) according to claim 7, **characterized by** comprising electronic control means (80) comprising in particular an electronic board (80), which are able to control preferably said ventilation means and said at least an internal UV light source (25) and preferably an air ionizer (70).

9. Air purification system (10) according to claim 8, **characterized by** comprising a control interface comprising preferably a plurality of buttons and in particular also a display for control preferably said electronic control means (80).

10. Household appliance comprising an air purification system (10) according to any one of the claims from 1 to 9, which is able to purify said air and to put again the same in said environment preferably domestic.

11. Filtering hood for a domestic environment in particular of a kitchen comprising an air purification system according to any one of the claims from 1 to 9, said filtering hood being preferably integrated with said air purification system (10) and being able to purify said air and to put again the same in said environment preferably domestic.

12. Air purification method preferably of a civil environment, in particular of a domestic environment, **characterized by** comprising a phase of a) illuminate at least a substance preferably antibacterial comprising titanium dioxide and silver ion with UV rays having a wavelength inferior to 400 nm obtaining at least an activated substance, and **characterized by** comprising a phase of b) force an air flow preferably at low speed on said at least an activated substance obtaining a purified air through photocatalysis.

13. Air purification method according to claim 12, **characterized by** comprising a phase of c) filtering and/or ionizing said purified air.

14. Air purification method according to claim 12 or 13, **characterized by** comprising a phase of d) put said purified air in said environment.
